# FASCICULE DE BREVET EUROPEEN

(11) **EP 3 442 632 B1**
(45) Date de publication et mention de la délivrance du brevet: **05.02.2020**
(21) Numéro de dépôt: 17723447.3
(22) Date de dépôt: 13.04.2017
(51) Int. Cl.: A61M 16/00, A61M 15/00

(54) **DISPOSITIF DE DISTRIBUTION DE PRODUIT FLUIDE SYNCHRONISE AVEC L'INHALATION**
VORRICHTUNG ZUR ABGABE EINES FLÜSSIGEN PRODUKTES MIT INHALATIONSSYNCHRONISATION
DEVICE FOR THE DISPENSING OF A FLUID PRODUCT SYNCHRONISED WITH INHALATION

(30) Priorité: 15.04.2016 FR 1653375
(43) Date de publication de la demande: 20.02.2019
(73) Titulaire: Aptar France SAS, 27110 Le Neubourg (FR)
(72) Inventeur: BROUET, Guillaume, 76000 Rouen (FR); PETIT, Ludovic, 27110 Vitot (FR)
(74) Mandataire: CAPRI
(86) Numéro de dépôt international: PCT/FR2017/050894
(87) Numéro de publication internationale: WO 2017/178767

(56) Documents cités:
- EP-A1- 0 441 643
- WO-A1-92/05824
- DE-U1-202004 021 188
- FR-A1- 2 775 668
- US-A- 5 060 643

## Description

La présente invention concerne un dispositif de distribution de produit fluide synchronisé avec l'inhalation, et plus particulièrement un dispositif d'inhalation du type aérosol synchronisé avec l'inhalation.

Les dispositifs actionnés par l'inhalation, désignés généralement par le terme B.A.I. (signifiant "Breath Actuated Inhaler"), sont bien connus dans l'état de la technique. L'avantage principal de ce type de dispositif est que la distribution du produit est synchronisée avec l'inhalation du patient, pour garantir une bonne distribution du produit dans les voies respiratoires. Ainsi, dans le domaine des dispositifs aérosol, c'est-à-dire ceux dans lesquels le produit est distribué à l'aide d'un gaz propulseur, de nombreux types de dispositifs de déclenchement par l'inhalation ont été proposés. Ces dispositifs présentent toutefois l'inconvénient de comporter un grand nombre de pièces, c'est-à-dire qu'ils sont compliqués et coûteux à fabriquer et à assembler, ce qui est évidemment désavantageux. Il est aussi difficile de trouver le bon équilibre entre un déclenchement fiable à chaque inhalation, sans que le seuil de déclenchement ne soit trop élevé, et une serrure suffisamment robuste pour empêcher un actionnement accidentel ou non souhaité. Or, si la serrure se déverrouille de manière accidentelle, le dispositif est actionné automatiquement et la dose est distribuée, même si l'utilisateur ne l'a pas souhaité.

Ainsi, plus que l'actionnement automatique du dispositif, ce qui est important pour obtenir une bonne distribution de la dose, c'est de réaliser cette distribution de manière synchronisée avec l'inhalation de l'utilisateur, même si l'actionnement ou le déclenchement proprement dit reste manuel.

Le document FR2775668 décrit un dispositif de l'état de la technique.

La présente invention a pour but de fournir un dispositif de distribution de produit fluide synchronisé avec l'inhalation qui ne reproduit pas les inconvénients susmentionnés.

La présente invention a aussi pour but de fournir un dispositif de distribution de produit fluide synchronisé avec l'inhalation qui améliore la fiabilité de fonctionnement, en garantissant un actionnement efficace à chaque inhalation.

La présente invention a aussi pour but de fournir un dispositif de distribution de produit fluide synchronisé avec l'inhalation qui minimise les risques d'actionnement accidentel ou non souhaité.

La présente invention a également pour but de fournir un dispositif de distribution de produit fluide synchronisé avec l'inhalation qui ne présente pas un seuil de déclenchement trop élevé, permettant ainsi à des personnes relativement faibles, telles que des personnes malades ou âgées, d'utiliser le dispositif de manière sûre et fiable.

La présente invention a également pour but de fournir un dispositif de distribution de produit fluide synchronisé avec l'inhalation qui soit simple et peu coûteux à fabriquer et à assembler.

La présente a donc pour objet un dispositif de distribution de produit fluide synchronisé avec l'inhalation, comportant un corps pourvu d'un embout buccal, un réservoir de produit contenant un produit fluide et un gaz propulseur étant monté axialement coulissant dans ledit corps, une valve doseuse comportant une soupape étant assemblée sur ledit réservoir pour distribuer sélectivement le produit fluide, ledit dispositif comportant:
- un élément d'actionnement déplaçable et/ou déformable entre une position de non actionnement, dans laquelle ladite valve doseuse ne peut pas être actionnée, et une position d'actionnement, dans laquelle ladite valve doseuse peut être actionnée,
- un système de déclenchement commandé par l'inhalation comportant un organe sensible à l'inhalation, déformable et/ou déplaçable sous l'effet de l'inhalation, ledit organe sensible à l'inhalation, lorsqu'il se déforme et/ou se déplace, déplaçant et/ou déformant ledit élément d'actionnement de sa position de non actionnement vers sa position d'actionnement,
- un compteur de doses électronique, et
- des moyens d'émission de signaux pour communiquer à distance notamment des informations relatives aux actionnements du dispositif.

Avantageusement, ledit élément d'actionnement est un élément de blocage qui, en position de non actionnement, coopère d'une part avec le corps et d'autre part avec le réservoir pour empêcher le déplacement axial dudit réservoir dans le corps.

Avantageusement, le dispositif comporte un élément déclencheur déplaçable et/ou déformable entre une position de verrouillage, dans laquelle il bloque ledit élément de blocage dans sa position de blocage, et une position de libération, dans laquelle il ne bloque pas ledit élément de blocage.

Avantageusement, ledit système de déclenchement commandé par l'inhalation comporte une membrane déformable définissant une chambre d'air déformable, ladite membrane déformable étant fixée audit élément déclencheur, ladite membrane déformable étant déformée lors de l'inhalation de telle sorte qu'elle déplace ledit élément déclencheur de sa position de blocage vers sa position de libération.

Avantageusement, ledit élément déclencheur est accessible manuellement par l'utilisateur pour pouvoir être déplacé manuellement vers sa position de libération même en l'absence d'inhalation.

Avantageusement, un organe d'actionnement est assemblé sur le réservoir du côté axialement opposé à ladite valve doseuse, ledit organe d'actionnement comportant un manchon creux déplaçable axialement par rapport audit réservoir entre une position de repos et une position chargée, un ressort étant disposé entre le fond du réservoir et le bord supérieur fermé dudit manchon creux, de sorte que lorsque l'utilisateur appuie manuellement sur ledit organe d'actionnement pour le déplacer vers sa position chargée, ledit ressort est comprimé, pour transmettre une force axiale F audit réservoir.

Avantageusement, une poignée d'actionnement latérale est montée pivotante sur le corps entre une position de repos et une position d'utilisation dans laquelle elle déplace axialement ledit organe d'actionnement dans sa position chargée.

Avantageusement, ledit corps comporte une ouverture reliant l'embout buccal avec l'intérieur du corps, ladite ouverture étant fermée en début d'inhalation par un clapet, de sorte que le flux d'inhalation est initialement principalement transmis au système de déclenchement par l'inhalation.

Avantageusement, ledit clapet est ouvert lorsque ledit élément de blocage se déplace vers sa position d'actionnement.

Avantageusement, ledit élément de blocage comporte une extension axiale dont une extrémité inférieure est fixée radialement et axialement par rapport audit corps et une extrémité supérieure coopère avec ledit réservoir en position de non actionnement.

Avantageusement, ledit système de déclenchement commandé par l'inhalation comporte un piston coulissant dans une chambre entre une position de repos et une position d'inhalation.

Avantageusement, ledit élément de blocage est assemblé sur une tige solidaire du piston, de sorte que lors de l'inhalation, ladite tige se déplace radialement déformant et/ou déplaçant ladite extension axiale vers sa position d'actionnement.

Avantageusement, ledit élément d'actionnement est un élément de verrouillage qui, en position de non actionnement, permet le déplacement axial de ladite soupape de la valve doseuse ensemble avec ledit réservoir dans le corps, empêchant l'actionnement de ladite valve doseuse lorsque ledit réservoir est déplacé axialement dans le corps sans inhalation.

Avantageusement, lors de l'inhalation, ledit élément de verrouillage est déplacé et/ou déformé de telle sorte qu'il bloque le déplacement axial de la soupape par rapport au corps.

Avantageusement, ledit système de déclenchement commandé par l'inhalation comporte un piston coulissant dans une chambre entre une position de repos et une position d'inhalation.

Avantageusement, ledit élément de verrouillage est solidaire d'une tige solidaire du piston, de sorte que lors de l'inhalation, ladite tige se déplace radialement déplaçant ledit élément de verrouillage vers sa position d'actionnement dans laquelle il empêche le déplacement axial de ladite soupape de la valve doseuse lorsque ledit réservoir est déplacé axialement dans le corps.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en section d'un dispositif de distribution de produit fluide, selon un premier mode de réalisation avantageux, en position de repos,
- la figure 2 est une vue schématique en section d'un dispositif de distribution de produit fluide, selon un autre mode de réalisation avantageux, en position de repos,
- la figure 3 est une vue similaire à celle de la figure 2, lorsque l'utilisateur tente d'actionner le dispositif sans inhalation,
- la figure 4 est une vue similaire à celle de la figure 3, lorsque l'utilisateur actionne le dispositif avec inhalation simultanée,
- la figure 5 est une vue schématique découpée d'un dispositif de distribution de produit fluide, selon encore un autre mode de réalisation avantageux, en position de repos,
- la figure 6 est une vue similaire à celle de la figure 5, en position de distribution,
- la figure 7 est une vue similaire à celle de la figure 5, illustrant une variante de réalisation,
- la figure 8 est une vue schématique en section d'un dispositif de distribution de produit fluide, selon encore un autre mode de réalisation avantageux, en position de repos,
- la figure 9 est une vue similaire à celle de la figure 8, après actionnement, et
- la figure 10 est une vue en perspective éclatée du dispositif des figures 8 et 9.

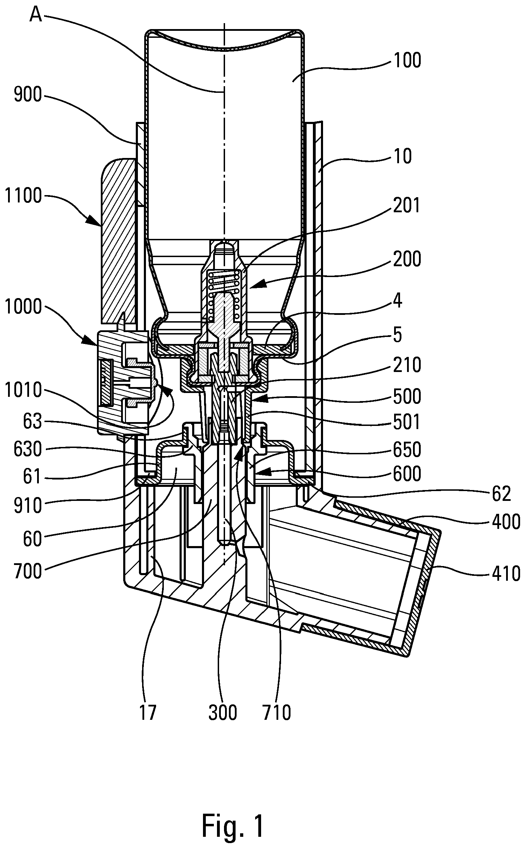

Dans la description, les termes "supérieur", "inférieur", "haut" et "bas" se réfèrent à la position du dispositif représentée notamment sur la figure 1. Les termes "axial" et "radial" se réfèrent à l'axe central vertical A représenté notamment sur la figure 1. Les termes "proximal" et "distal" se réfèrent à l'embout buccal.

L'invention s'applique plus particulièrement à des dispositifs d'inhalation du type à valve aérosol pour une distribution orale, comme cela sera décrit plus en détail ci-après, mais elle pourrait aussi s'appliquer à d'autres types de dispositifs d'inhalation, par exemple du type nasal.

Les figures représentent plusieurs modes de réalisation avantageux de l'invention, mais il est entendu l'une ou plusieurs des pièces constitutives décrites ci-après pourrai(en)t être réalisée(s) différemment tout en procurant des fonctions similaires voire identiques.

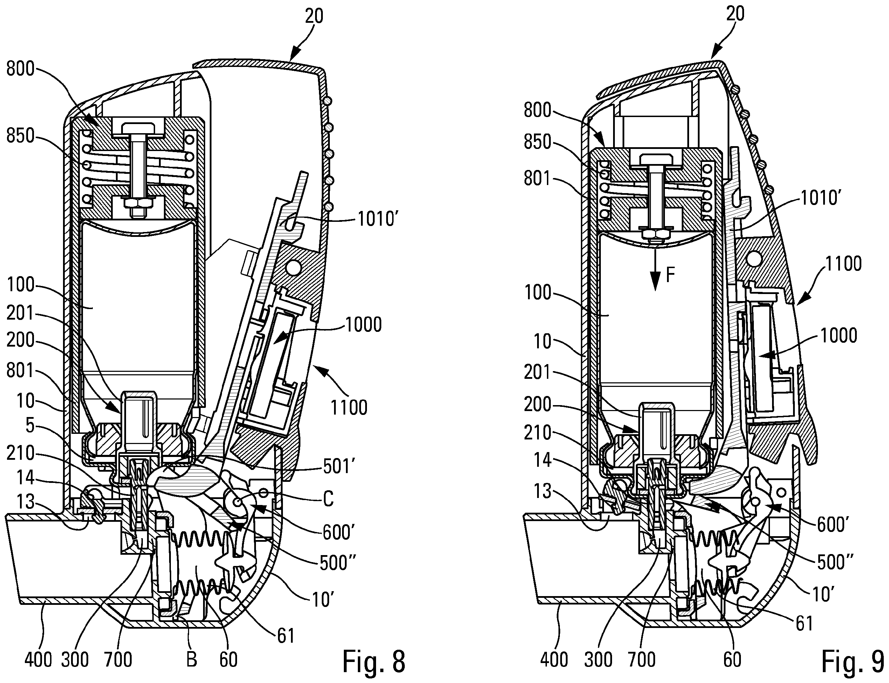

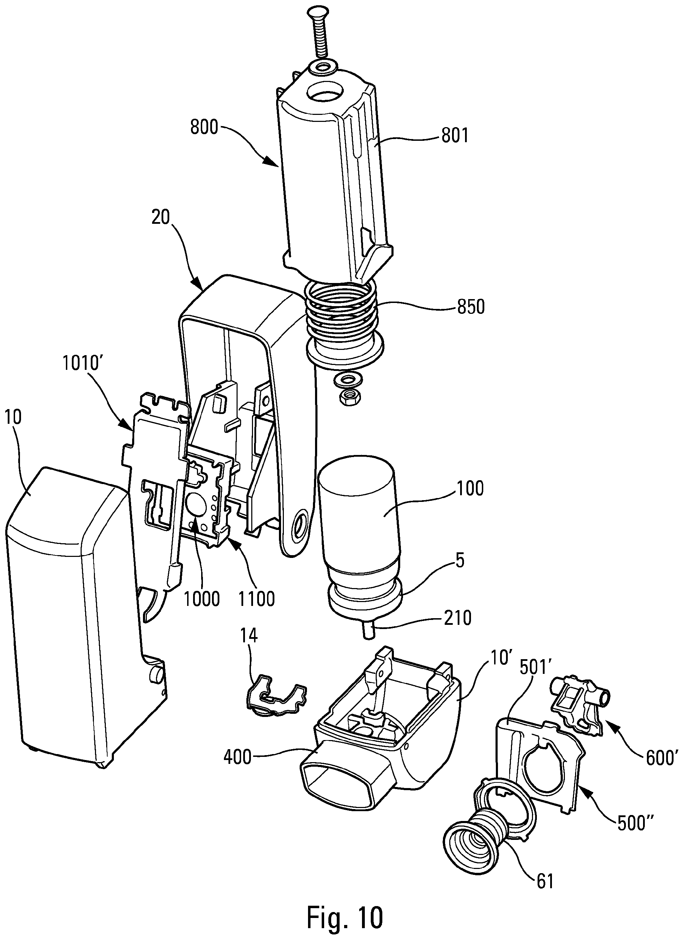

En référence aux dessins, le dispositif comporte un corps principal 10 pourvu d'un embout buccal 400. Cet embout buccal 400 définit un orifice de distribution à travers lequel l'utilisateur va inhaler lors de l'utilisation du dispositif. L'embout buccal 400 peut être réalisé d'une pièce avec le corps 10, comme sur les figures 1 à 7, ou être formé sur une partie inférieure de corps 10' qui se fixe audit corps principal 10, comme sur les figures 8 à 10. Un capot de protection amovible 410 peut être prévu sur ledit embout buccal 400, notamment lors des périodes de stockage, que l'utilisateur va retirer avant utilisation. La figure 1 montre un tel capot de protection, qui pourrait être de forme quelconque.

Le corps principal 10 contient un réservoir 100, contenant le produit à distribuer et un gaz propulseur, tel qu'un gaz du type HFA, une valve doseuse 200 étant montée sur ledit réservoir 100 pour distribuer sélectivement le produit. La valve doseuse 200 comporte un corps de valve 201 et une soupape 210 axialement déplaçable lors de l'actionnement par rapport audit corps de valve 201, et donc par rapport audit réservoir 100. Cette valve doseuse 200 peut être d'un type quelconque approprié. Elle peut être fixée au réservoir 100 par un élément de fixation, de préférence une capsule sertie 5, de préférence avec interposition d'un joint de col 4.

Avantageusement, lors de l'actionnement, la soupape 210 est fixe par rapport au corps 10, et c'est le réservoir 100 qui est déplacé axialement par rapport au corps 10 entre une position distale, qui est la position de repos, et une position proximale.

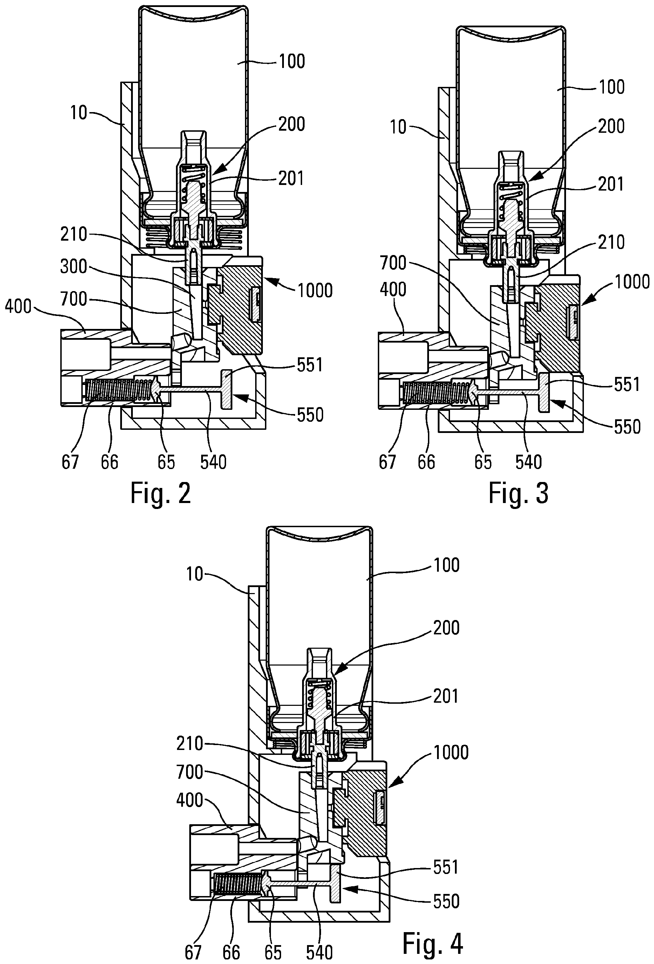

L'orifice de sortie de la soupape 210 de ladite valve doseuse 200 est relié via un canal 300 audit embout buccal 400, à travers lequel l'utilisateur inhale le produit distribué. De manière connue, ladite soupape 210 est reçue dans un puits de soupape 700 qui définit au moins partiellement ledit canal 300. Dans les modes de réalisation des figures 1 et 8 à 10, le puits de soupape 700 est formé d'une pièce avec le corps 10 ou la partie inférieure de corps 10', alors que dans les modes de réalisations des figures 2 à 7, ledit puits de soupape 700 est axialement déplaçable par rapport audit corps 10.

Selon l'invention, le dispositif comporte un élément d'actionnement 500, 500', 500", 550 déplaçable et/ou déformable entre une position de non actionnement, dans laquelle ladite valve doseuse 200 ne peut pas être actionnée, et une position d'actionnement, dans laquelle ladite valve doseuse 200 peut être actionnée. En position de repos, ledit élément d'actionnement 500, 500', 500", 550 est en position de non actionnement, et c'est l'inhalation de l'utilisateur à travers l'embout buccal 400 qui déplace et/ou déforme ledit élément d'actionnement 500, 500', 500", 550 vers sa position d'actionnement. En d'autres termes, tant que l'utilisateur n'a pas inhalé, l'actionnement de la valve doseuse 200 est impossible, et ce n'est que lorsqu'il inhale qu'il peut actionner ladite valve doseuse 200, avantageusement par appui manuel sur le fond du réservoir 100.

Comme cela sera décrit plus en détails ci-après, l'élément d'actionnement peut être un élément de blocage 500, 500', 500" qui, en position de non actionnement, empêche le déplacement axial du réservoir 100 dans le corps 10. Lors de l'inhalation, cet élément de blocage 500, 500', 500" est déplacé et/ou déformé de telle sorte qu'il ne bloque plus le déplacement axial du réservoir 100 dans le corps 10. Ainsi, après inhalation, un tel déplacement axial du réservoir 100 provoque l'actionnement de la valve doseuse 200 et la distribution d'une dose de produit synchronisée avec cette inhalation.

En variante, comme cela sera décrit en référence aux figures 2 à 4, l'élément d'actionnement peut être un élément de verrouillage 550 qui, en position de non actionnement, permet le déplacement axial de la soupape 210 de la valve doseuse 200 ensemble avec réservoir 100 dans le corps 10, empêchant par-là l'actionnement de ladite valve doseuse 200 lorsque ledit réservoir 100 est déplacé axialement dans le corps 10 sans inhalation. Lors de l'inhalation, cet élément de verrouillage 550 est déplacé et/ou déformé de telle sorte qu'il bloque le déplacement axial de la soupape 210 par rapport au corps 10. Ainsi, après inhalation, un déplacement axial du réservoir 100 provoque l'actionnement de la valve doseuse 200 et la distribution d'une dose de produit synchronisée avec cette inhalation.

Ainsi, dans les diverses variantes décrites ci-dessus, en l'absence d'inhalation, il n'y a aucun risque qu'une dose de produit actif soit perdue par un actionnement malencontreux ou incomplet dans lequel l'utilisateur n'exercerait pas d'inhalation. L'actionnement de la valve 200 et l'expulsion d'une dose de produit fluide ne sont donc possibles que si l'utilisateur inhale et que simultanément il appuie sur le réservoir 100 pour actionner la valve 200.

Le dispositif comporte un système de déclenchement commandé par l'inhalation de l'utilisateur, qui est destiné à déplacer et/ou déformer ledit élément d'actionnement 500, 500', 500", 550 de sa position de non actionnement vers sa position d'actionnement, lorsque l'utilisateur inhale à travers l'embout buccal 400.

Ce système de déclenchement comporte un organe sensible à l'inhalation 60, 61, 65, 66 déformable et/ou déplaçable sous l'effet de l'inhalation, cet organe sensible à l'inhalation 60, 61, 65, 66 étant adapté, lorsqu'il se déforme et/ou se déplace, à déplacer et/ou déformer ledit élément d'actionnement 500, 500', 500", 550 de sa position de non actionnement vers sa position d'actionnement.

Comme cela sera décrit plus en détails ci-après, l'organe sensible à l'inhalation peut être réalisé sous la forme d'une membrane déformable 61 définissant une chambre d'air déformable 60, par exemple un soufflet ou une poche déformable.

En variante, comme cela sera décrit en référence aux figures 2 à 7, l'organe sensible à l'inhalation peut être réalisé sous la forme d'un piston 65, de préférence cylindrique, coulissant dans une chambre 66, de préférence cylindrique, non déformable.

Selon l'invention, le dispositif embarque des modules électroniques.

En particulier, il est prévu un compteur de doses électronique 1000. Ce compteur 1000 peut notamment détecter les déplacements du réservoir 100, par exemple au moyen d'un capteur de contact 1010. En variante, le compteur 1000 pourrait être relié à un capteur, notamment un capteur à membrane, qui détecte la distribution de la dose de produit fluide, par exemple dans le puits de soupape 700. D'autres moyens d'actionner le compteur électronique 1000 sont aussi possibles, par exemple la détection du déplacement de la soupape 210 de la valve doseuse par rapport au corps de valve 201.

Le dispositif comporte aussi des moyens d'émission de signaux 1100 pour communiquer à distance notamment des informations relatives aux actionnements du dispositif. En particulier, le corps 10 peut comporter un module d'émission de signaux, pour communiquer à distance avec une base quelconque. Des moyens d'alimentation appropriés sont avantageusement prévus.

Avantageusement, le module électronique peut comprendre notamment une carte comportant un commutateur électrique qui envoie une impulsion. Le module peut également comporter un afficheur et/ou utiliser une connexion Bluetooth ou Wifi pour envoyer les informations sur un périphérique annexe. Des capteurs appropriés peuvent être prévus, tel que des capteurs de débit et/ou de pression, pour détecter divers paramètres du flux d'inhalation.

Associés à un compteur de dose 1000 qui compte chaque dose effectivement émise et au dispositif de synchronisation avec l'inhalation de l'invention, ces moyens d'émission de signaux 1100 permettent de transmettre de manière absolument fiable chaque distribution de dose, par exemple à un médecin ou toute autre personne souhaitant surveiller l'utilisation du dispositif d'inhalation par l'utilisateur. Le dispositif de synchronisation avec l'inhalation garantit que l'utilisateur inhale à chaque fois qu'il actionne le dispositif, et le compteur enregistre chaque dose distribuée, ainsi que différents paramètres associés, tel que l'horodatage de chaque distribution et les caractéristiques de l'inhalation. Le médecin peut ainsi connaitre très exactement les conditions d'utilisation du dispositif par l'utilisateur.

Selon un premier mode de réalisation, représenté sur la figure 1, la position de non actionnement correspond à une position de blocage du réservoir 100 dans le corps 10. Dans cette position de blocage, le réservoir 100 est empêché de se déplacer par ledit élément d'actionnement, qui ne sera libéré qu'au moment de l'inhalation.

L'élément d'actionnement, formant élément de blocage, est ici avantageusement formé par une bague de blocage 500, comportant au moins une, de préférence trois pattes axiales de blocage 501, qui sont élastiquement déformable radialement vers l'extérieur. Cette bague de blocage 500 est fixée, notamment encliquetée, sur le réservoir 100, notamment sur la capsule 5 qui fixe la valve doseuse 200 sur le réservoir 100. Lesdites pattes de blocage 501 s'appuient en position de repos sur un épaulement radial 710 dudit puits de soupape 700. Cet épaulement est de préférence incliné vers le bas et radialement vers l'extérieur, de sorte que lorsque le réservoir 100 coulisse axialement dans le corps 10 lors de l'actionnement, lesdites pattes axiales de blocage 501 glissent sur ledit épaulement 710 incliné, ce qui les déforme radialement vers l'extérieur.

Un élément déclencheur 600 est monté coulissant axialement autour dudit puits de soupape 700 entre une position de blocage, dans laquelle il bloque ladite bague de blocage 500 dans sa position de non actionnement, et une position de libération, dans laquelle il ne bloque plus ladite bague de blocage 500. En particulier, dans le mode de réalisation de la figure 1, ledit élément déclencheur 600, en position de blocage, coopère avec lesdites pattes de blocage 501, empêchant toute déformation radiale vers l'extérieur desdites pattes de blocage 501. Ainsi, lorsque ledit élément déclencheur 600 est en position de blocage, il empêche la déformation radiale vers l'extérieur desdites pattes de blocage 501, qui par conséquent restent axialement bloquées par ledit épaulement 710 du puits de soupape 700, ce qui bloque le déplacement axial du réservoir 100 et donc l'actionnement de la valve doseuse 200. Eventuellement, des moyens de glissement, tels que des billes, pourraient être interposées entre le puits de soupape 700 et l'élément déclencheur 600, pour faciliter le coulissement de ce dernier lors de l'inhalation.

L'élément déclencheur 600 comporte avantageusement un manchon central creux 650 qui coulisse axialement autour du puits de soupape 700. Deux pattes axiales (non représentées), diamétralement opposées, et chacune reliée audit manchon central 650 peuvent être prévues pour coopérer chacune avec une ouverture respective du corps 10, pour substantiellement obturer ces ouvertures dans la position de blocage, et pour substantiellement ouvrir ces ouvertures dans la position de libération. Ces ouvertures étant fermées en début d'inhalation, le flux d'inhalation est initialement principalement transmis au système de déclenchement par l'inhalation, dans cet exemple la chambre d'air déformable 60. Ceci permet d'optimiser ce déclenchement par l'inhalation. Lorsque l'élément déclencheur 600 s'est déplacé axialement vers sa position de libération sous l'effet de l'inhalation, et donc lorsque l'utilisateur peut actionner la valve doseuse 200 pour distribuer une dose de produit fluide, les pattes axiales ouvrent lesdites ouvertures du corps 10, ce qui génère un appel d'air, et augmente donc le flux d'inhalation. Ceci optimise la synchronisation entre la distribution de la dose et l'inhalation de l'utilisateur, et favorise une bonne distribution de la dose dans les poumons de l'utilisateur.

Avantageusement, lesdites pattes axiales peuvent être accessibles de l'extérieur à travers lesdites ouvertures. Ceci permet en cas de nécessité de déplacer manuellement l'élément déclencheur 600, pour pouvoir réaliser un actionnement de la valve doseuse 200 même sans inhalation, par exemple si la personne devant recevoir la dose de produit fluide est incapable de réaliser une inhalation suffisante. Il s'agit donc d'une sécurité. En variante pour cette sécurité, on pourrait prévoir une extension axiale (non représentée) solidaire de l'élément déclencheur 600, s'étendant par exemple latéralement du réservoir 100, et accessible par l'utilisateur depuis l'extérieur du corps 10.

En variante, l'élément déclencheur 600 peut ne pas comporter les pattes axiales, et le corps peut ne pas comporter les ouvertures 13. Dans ce cas, le flux d'inhalation pourrait s'écouler axialement dans le corps 10, autour du puits de soupape 700, ce qui pourrait être favorisé par un manchon central 650 pourvu de découpes axiales permettant le passage du flux d'air.

Dans le mode de réalisation de la figure 1, l'organe sensible à l'inhalation est réalisé sous la forme d'une chambre d'air déformable 60. Avantageusement, cette chambre d'air comprend une membrane déformable 61 qui est reliée d'une part au corps 10 et d'autre part audit élément déclencheur 600. Avantageusement, un manchon porte-membrane 900 est disposé fixement dans le corps 10, avec un épaulement inférieur 910 qui coince un premier bord 62 de la membrane 61 contre une partie 17 du corps 10. Le second bord 63 de la membrane 61 peut être fixé dans une gorge 630 de l'élément déclencheur 600, avantageusement formée sur le manchon central creux 650 de celui-ci.

Lors de l'inhalation, la membrane déformable 61 se déforme et/ou se contracte sous l'effet de la dépression générée par l'inhalation, provoquant le déplacement de l'élément déclencheur 600 de sa position de blocage vers sa position de libération. Ceci permet alors la déformation radiale desdites pattes de blocage 501 et donc le déplacement de ladite bague de blocage 500 formant l'élément d'actionnement de sa position de non actionnement vers sa position d'actionnement. La figure 1 montre une membrane déformable 61 réalisée sous la forme d'une poche ou d'un diaphragme. Bien entendu, d'autres formes sont aussi envisageables, par exemple un soufflet comme représenté sur les figures 8 à 10.

La valve 200 n'est donc actionnée qu'au moment de l'inhalation, de sorte que la dose de produit fluide est expulsée hors de l'orifice de distribution simultanément à l'inhalation. La distribution de la dose est comptée par le compteur 1000, et les informations relatives à la prise de dose, tel que par exemple l'horodatage et les paramètres du flux d'inhalation, sont transmis par les moyens d'émission 1100.

Lorsque l'utilisateur souhaite utiliser le dispositif, il place l'embout buccal 400 dans sa bouche, et appuie manuellement sur le fond du réservoir 100, c'est-à-dire la surface supérieure dudit réservoir 100 dans la position de la figure 1. Le réservoir 100 va alors se trouver bloqué et empêché de coulisser dans le corps 10 par les pattes de blocage 501 de la bague de blocage 500, qui s'appuient sur l'épaulement 710 du puits de soupape 700. Eventuellement, le réservoir 100 peut effectuer une petite course avant d'être bloqué, cette petite course initiale étant toutefois insuffisante pour actionner la valve doseuse 200.

Lorsque l'utilisateur inhale à travers l'embout buccal 400, il va déformer la membrane déformable 61, ce qui va faire coulisser l'élément déclencheur 600 fixé à ladite membrane déformable 61. Ce déplacement de l'élément déclencheur 600 sur le puits de soupape 700 va libérer radialement les pattes 501 de la bague de blocage 500. Sous l'effet de la force axiale transmise par le réservoir 100, générée par l'utilisateur qui appuie sur le fond dudit réservoir 100, les pattes axiales 501 vont pouvoir se déformer radialement vers l'extérieur, et ainsi passer par-dessus ledit épaulement 710, pour permettre le coulissement du réservoir 100 vers sa position de distribution, et donc l'actionnement de la valve 200.

En fin d'inhalation, l'élément déclencheur 600 est ramené vers le haut par l'élasticité de la membrane 61.

Lorsque l'utilisateur relâche sa pression sur le fond du réservoir 100, celui-ci revient en direction de la position de repos sous l'effet du ressort de rappel de la valve 200, et simultanément la soupape 210 de la valve doseuse revient en position de repos, en chargeant une nouvelle dose de produit fluide dans la chambre de valve. Le dispositif est alors prêt pour une autre utilisation.

Les figures 2 à 4 illustrent un autre mode de réalisation de l'invention. Ici, l'élément d'actionnement est un élément de verrouillage 550 qui, en position de non actionnement, permet le déplacement axial de la soupape 210 de la valve doseuse 200 ensemble avec le réservoir 100 dans le corps 10, empêchant l'actionnement de ladite valve doseuse 200 lorsque ledit réservoir 100 est déplacé axialement dans le corps 10 sans inhalation. Lors de l'inhalation, cet élément de verrouillage 550 est déplacé et/ou déformé de telle sorte qu'il bloque le déplacement axial de la soupape 210 par rapport au corps 10. Ainsi, après inhalation, un déplacement axial du réservoir 100 provoque l'actionnement de la valve doseuse 200 et la distribution d'une dose de produit synchronisée avec cette inhalation.

L'organe sensible à l'inhalation est réalisé sous la forme d'un piston 65 coulissant dans une chambre 66, entre une position de repos et une position d'inhalation. La chambre 66 est avantageusement formée dans l'embout buccal 400. Ledit piston 65 est relié audit élément de verrouillage 550, avantageusement par une tige 540. En particulier, comme visible sur les figures 2 à 4, l'élément de verrouillage 550 peut être formé à l'extrémité de ladite tige 540 opposée audit piston 65, et comprend une projection axiale 551. Un ressort 67, disposé avantageusement dans la chambre 66, est adapté à ramener ledit piston 65 vers sa position de repos lorsqu'il n'y a plus d'inhalation à travers l'embout buccal 400.

En position de non actionnement, ladite projection est décalée radialement par rapport au puits de soupape 700, de sorte que celui-ci peut se déplacer axialement dans le corps 10, ensemble avec la soupape 210 de la valve doseuse 200 et le réservoir 100. Ainsi, dans cette position de non actionnement, la soupape 210 ne se déplace par rapport au réservoir 100, et la valve doseuse 200 n'est donc pas actionnée.

Lorsque l'utilisateur inhale à travers l'embout buccal 400, le piston 65 se déplace radialement (par rapport à l'axe A de déplacement du réservoir 100 dans le corps 10) dans la chambre 66 sous l'effet de la dépression crée par l'inhalation. La projection 551 se déplace donc également radialement, et vient se positionner sous ledit puits de soupape 700, formant ainsi une butée au déplacement axial vers le bas dudit puits de soupape. De ce fait, la pression exercée par l'utilisateur sur le fond du réservoir 100 va déplacer celui-ci axialement vers le bas dans le corps, et le puits de soupape 700, maintenant axialement fixe par rapport au corps 10, va donc bloquer la soupape 210 de la valve doseuse axialement par rapport au corps 10, de sorte qu'elle va s'enfoncer dans le corps de valve, provoquant ainsi l'actionnement de la valve doseuse 200 et la distribution d'une dose de produit fluide.

Bien entendu, dans ce mode de réalisation où le réservoir 100 est déplaçable axialement dans le corps 10 aussi bien en position d'actionnement que de non actionnement de l'élément d'actionnement 550, le compteur de dose 1000 ne peut pas mesurer ce déplacement axial du réservoir 100. On privilégiera dans ce cas des capteurs détectant la distribution du produit fluide, notamment dans le puits de soupape 700, ou des capteurs détectant le déplacement de la soupape 210 de la valve doseuse 200 par rapport au corps de valve 201.

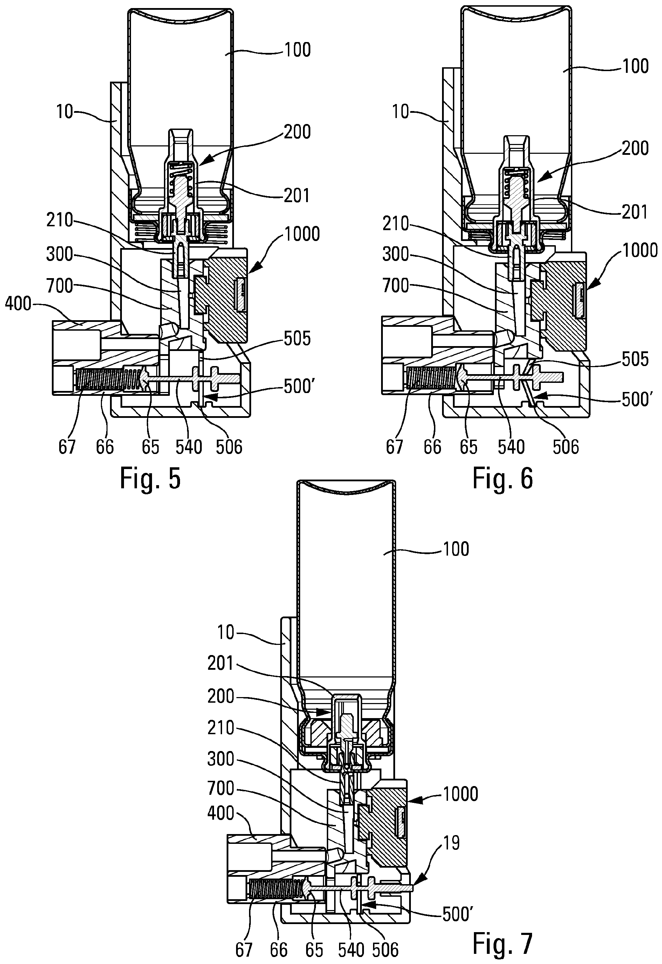

Les figures 5 à 7 illustrent encore un autre mode de réalisation. Ici, le système de déclenchement par l'inhalation est similaire à celui décrit ci-dessus en référence aux figures 2 à 4, avec un piston 65 coulissant radialement dans une chambre 66 de l'embout buccal 400.

Ici, l'élément d'actionnement est à nouveau réalisé sous la forme d'un élément de blocage 500', qui, en position de non actionnement, empêche le déplacement axial du réservoir 100 dans le corps 10. Lors de l'inhalation, cet élément de blocage 500' est déplacé et/ou déformé de telle sorte qu'il ne bloque plus le déplacement axial du réservoir 100 dans le corps 10. Ainsi, après inhalation, un tel déplacement axial du réservoir 100 provoque l'actionnement de la valve doseuse 200 et la distribution d'une dose de produit synchronisée avec cette inhalation.

Cet élément de blocage 500' peut notamment être assemblé sur ladite tige 540 solidaire du piston 65, et comporter une extension axiale 505 qui, en position de non actionnement s'étend axialement dans le corps pour coopérer avec et bloquer axialement ledit réservoir 100. Lorsque l'utilisateur inhale, la tige 540 se déplace radialement vers la gauche (dans l'orientation des figures 5 à 7), ce qui va provoquer la déformation de ladite extension axiale 505, et donc libérer le déplacement axial du réservoir 100. Dans l'exemple représenté, le puits de soupape 700 est monté mobile dans le corps 10, mais il pourrait aussi être fixe.

Avantageusement, l'extrémité inférieure 506 de ladite extension axiale 505 est fixée radialement et axialement par rapport au corps 10. Ainsi, lorsque la tige 540 se déplace radialement, elle tire radialement sur ladite extension axiale, qui se déforme, par exemple se plie ou pivote, de sorte que l'extrémité supérieure se désengage du réservoir 100 et libère celui-ci pour son déplacement axial. Bien entendu, l'élément de blocage 500' pourrait avoir toute autre forme appropriée. En particulier, on peut envisager l'utilisation d'une genouillère articulée.

La figure 7 représente une variante de réalisation, dans laquelle la tige 540 est accessible de l'extérieur du corps 10 à travers une ouverture 19 dudit corps 10. Ceci permet en cas de nécessité de déplacer manuellement l'élément de blocage 500', pour pouvoir réaliser un actionnement de la valve doseuse 200 même sans inhalation, par exemple si la personne devant recevoir la dose de produit fluide est incapable de réaliser une inhalation suffisante. Il s'agit donc d'une sécurité. Il est à noter que cette sécurité pourrait aussi s'adapter au mode de réalisation des figures 2 à 4.

Dans l'exemple des figures 8 à 10, un organe d'actionnement 800 est assemblé sur l'extrémité supérieure du réservoir 100, opposée axialement à ladite valve doseuse 200. Cet organe d'actionnement 800 comporte un manchon creux 801 disposé dans le corps 10 autour du réservoir 100, avec un ressort 850 disposé entre le fond du réservoir 100 et le bord supérieur fermé dudit manchon creux 801. Le manchon creux 801 est déplaçable axialement par rapport audit réservoir 100 entre une position de repos et une position chargée. Ainsi, lorsque l'utilisateur souhaite déplacer le réservoir 100 axialement dans le corps 10, pour actionner la valve doseuse 200, il appuie sur ledit organe d'actionnement 800. Ceci va déplacer axialement ledit manchon creux 801 vers sa position chargée et ainsi comprimer ledit ressort 850, qui va donc transmettre une force axiale F audit réservoir 100 qui est sensiblement la même à chaque actionnement. Tant que l'utilisateur maintient son appui sur ledit organe d'actionnement 800, ledit ressort 850 est comprimé et sollicite axialement ledit réservoir 100 vers sa position d'actionnement.

Dans la variante des figures 8 à 10 une poignée d'actionnement latérale 20 est montée pivotante sur le corps 10. Cette poignée, lorsque déplacée de sa position de repos, visible sur la figure 8, vers sa position d'utilisation, visible sur la figure 9, vient déplacer axialement ledit organe d'actionnement 800 pour comprimer le ressort 850. Ceci peut notamment se faire au moyen d'une came sur la poignée 20 coopérant avec un profil complémentaire sur l'organe d'actionnement 800. Tant que l'utilisateur maintient son appui sur ladite poignée, ledit ressort 850 est comprimé et sollicite axialement ledit réservoir 100 vers sa position d'actionnement. Avantageusement, ladite poignée 20 comporte un organe de sollicitation qui sollicite ladite poignée 20 vers sa position de repos. Ainsi, lorsque l'utilisateur relâche sa pression sur la poignée 20, celle-ci revient automatiquement vers sa position de repos. Ceci permet d'éviter le risque qu'après actionnement de la valve doseuse 200, cette dernière reste en position actionnée, ce qui pourrait avoir pour conséquence que la chambre de la valve se remplit d'air et que la dose suivante est incomplète voire que la valve fuit. Ceci est une des problématiques rencontrées actuellement par les dispositifs existants sur le marché.

Bien entendu, cette poignée 20 n'est pas nécessaire, ni d'ailleurs l'organe d'actionnement 800, l'utilisateur pouvant directement appuyer sur le fond du réservoir, comme dans les modes de réalisation précédemment décrits.

L'actionnement de la valve 200 et l'expulsion d'une dose de produit fluide ne sont donc possibles que si l'utilisateur inhale et que simultanément il appuie axialement sur le réservoir 100 pour actionner la valve 200. Comme décrit précédemment, cet appui axial sur le réservoir 100 peut se faire par l'intermédiaire de l'organe d'actionnement 800 qui comprime le ressort 850. En variante, l'utilisateur pourrait directement appuyer sur le fond du réservoir 100. Dans la variante des figures 8 à 10, c'est la poignée 20 qui va générer cet appui axial, également via l'organe d'actionnement 800 dans l'exemple représenté. Enfin, on pourrait aussi utiliser un système d'actionnement automatique, qui appliquerait cet appui axial sur le réservoir 100 indépendamment de l'utilisateur.

Dans le mode de réalisation des figures 8 à 10, l'élément d'actionnement est un élément de blocage 500" qui, en position de non actionnement, empêche le déplacement axial du réservoir 100 dans le corps 10.

L'élément de blocage 500" est avantageusement monté pivotant autour d'un axe B sur le corps 10 ou sur la partie de corps 10' entre une position de blocage et une position d'actionnement. Dans l'exemple représenté, ledit axe B passe par un bord inférieur dudit élément de blocage 500".

L'élément de blocage 500" comporte au moins une, de préférence deux extensions de blocage 501', qui coopèrent en position de blocage avec le réservoir 100 (avantageusement avec la capsule de sertissage 5).

L'élément de blocage 500" est retenu en position de blocage par un élément déclencheur 600'. Cet élément déclencheur 600' est monté pivotant autour d'un axe C sur le corps 10, sur la partie de corps 10' ou sur la poignée 20, entre une position de verrouillage, dans laquelle il bloque ledit élément de blocage 500" dans sa position de blocage, et une position de libération, dans laquelle il ne bloque plus ledit élément de blocage 500". Dans l'exemple représenté, ledit axe C passe environ au milieu dudit élément déclencheur 600'. Avantageusement, les axes B et C sont parallèles.

L'élément de blocage 500" et l'élément déclencheur 600' définissent ensemble une serrure. En particulier, ledit élément déclencheur 600' comporte un épaulement de verrouillage qui, en position de verrouillage, coopère avec une projection de l'élément de blocage 500", empêchant le pivotement dudit élément de blocage 500" hors de sa position de blocage. Ainsi, lorsque ledit élément déclencheur 600' est en position de verrouillage, il empêche le déplacement de l'élément de blocage 500" vers sa position d'actionnement, ce qui bloque le déplacement axial du réservoir 100 et donc l'actionnement de la valve doseuse 200.

Grâce à ce système d'effort de la serrure, l'effort nécessaire pour faire pivoter l'élément déclencheur 600' est très faible et peut être généré par la membrane déformable 61, qui permet de transformer la dépression générée par l'inhalation en force de déverrouillage.

Avantageusement, la partie inférieure de corps 10' comporte une ouverture 13 reliée avec l'intérieur du corps 10. Cette ouverture 13 est fermée au repos et en début d'inhalation par un clapet 14, de sorte que le flux d'inhalation est initialement principalement transmis au système de déclenchement par l'inhalation, dans cet exemple la chambre d'air déformable 60. Ceci permet d'optimiser ce déclenchement par l'inhalation. Lorsque l'élément de blocage 500" s'est déplacé vers sa position d'actionnement sous l'effet de l'inhalation, et donc lorsque l'utilisateur peut actionner la valve doseuse 200 pour distribuer une dose de produit fluide, ledit élément de blocage 500" déplace ledit clapet 14 vers une position ouverte. Lorsque lesdites ouvertures 13 sont ainsi ouvertes, un appel d'air est généré, ce qui permet d'augmenter le flux d'inhalation. Ceci optimise la synchronisation entre la distribution de la dose et l'inhalation de l'utilisateur, et favorise une bonne distribution de la dose dans les poumons de l'utilisateur.

Avantageusement, l'élément déclencheur 600' peut être accessible de l'extérieur du corps 10 et/ou de la partie inférieure de corps 10'. Ceci permet en cas de nécessité de déplacer manuellement l'élément déclencheur 600', pour pouvoir réaliser un actionnement de la valve doseuse 200 même sans inhalation, par exemple si la personne devant recevoir la dose de produit fluide est incapable de réaliser une inhalation suffisante. Il s'agit donc d'une sécurité.

Dans le mode de réalisation représenté sur les figures 8 à 10, l'organe sensible à l'inhalation est réalisé sous la forme d'une chambre d'air déformable 60. Avantageusement, cette chambre d'air comprend une membrane déformable 61 qui est reliée d'une part à ladite partie inférieure de corps 10' et d'autre part audit élément déclencheur 600'. Avantageusement, comme visible sur les figures, la membrane 61 a une forme de soufflet et forme une chambre sensiblement étanche. D'autres formes sont possibles, notamment une simple poche ou diaphragme. Un plot peut fixer ladite membrane 61 à un orifice dudit élément déclencheur 600'.

Lors de l'inhalation, la membrane déformable 61 se déforme et/ou se contracte sous l'effet de la dépression générée par l'inhalation, provoquant le déplacement de l'élément déclencheur 600' de sa position de verrouillage vers sa position de libération. Ceci permet d'ouvrir la serrure définie entre l'élément de blocage 500" et l'élément déclencheur 600', et donc le déplacement dudit élément de blocage 500" de sa position de blocage vers sa position d'actionnement.

La valve 200 n'est donc actionnée qu'au moment de l'inhalation, de sorte que la dose de produit fluide est expulsée hors de l'orifice de distribution simultanément à l'inhalation.

Avantageusement, l'organe d'actionnement 800 comporte une patte de blocage (non représentée), pouvant coopérer en position de repos avec ledit élément déclencheur 600', pour l'empêcher de se déplacer vers sa position de libération. Ainsi, si l'utilisateur inhale sans avoir exercé l'appui axial sur le réservoir 100, la serrure ne sera pas débloquée, puisque l'élément déclencheur 600' ne peut pas pivoter. La chambre d'air 60 étant sensiblement étanche, et le clapet 14 étant fermé sur l'ouverture 13, l'utilisateur se rend compte très vite qu'il ne peut inhaler correctement à travers l'embout buccal 400, ce qui lui rappelle qu'il faut d'abord exercer l'appui axial sur le réservoir 100 avant d'inhaler. Lorsque l'utilisateur appuie sur l'organe d'actionnement 800, il déplace axialement le manchon 801 par rapport au réservoir 100, qui lui est bloqué par l'élément de blocage 500, ce qui comprime le ressort 850. Une inhalation provoquera alors un pivotement de l'élément déclencheur 600, et donc l'actionnement du dispositif, comme expliqué précédemment.

Lorsque l'utilisateur souhaite utiliser le dispositif, il place l'embout buccal 400 dans sa bouche, et exerce manuellement un appui axial sur le fond du réservoir 100, c'est-à-dire la surface supérieure dudit réservoir 100 dans la position des figures. Le réservoir 100 est bloqué et empêché de coulisser axialement dans le corps 10 par les extensions de blocage 501 de l'élément de blocage 500". Parallèlement, le blocage de l'élément déclencheur 600' est supprimé par le déplacement axial de l'organe d'actionnement 800.

Lorsque l'utilisateur inhale à travers l'embout buccal 400, il va déformer la membrane déformable 61, ce qui va faire pivoter l'élément déclencheur 600' fixé à ladite membrane déformable 61. Ce déplacement de l'élément déclencheur 600' va libérer la serrure formée entre l'élément déclencheur 600' et l'élément de blocage 500". Sous l'effet de la force axiale transmise par le réservoir 100, générée par l'appui axial sur le fond dudit réservoir 100, l'élément de blocage 500" va pivoter permettant le coulissement axial du réservoir 100 dans le corps 10 vers sa position de distribution, et donc l'actionnement de la valve 200. Parallèlement, l'élément de blocage 500" va ouvrir le clapet 14. '.

En fin d'inhalation, lorsque l'utilisateur relâche sa pression sur le fond du réservoir 100, celui-ci remonte axialement dans le corps en direction de sa position de repos sous l'effet du ressort de rappel de la valve 200, et simultanément la soupape 210 de la valve doseuse revient en position de repos, en chargeant une nouvelle dose de produit fluide dans la chambre de valve. L'élément déclencheur 600' est ramené dans sa position initiale par l'élasticité de la membrane 61 et/ou par l'organe d'actionnement 800 qui revient vers sa position de repos. L'élément de blocage 500" revient dans sa position de blocage, avantageusement via à un élément élastique, tel qu'un ressort ou un élément en élastomère (non représenté).

Le dispositif est alors prêt pour une autre utilisation.

En particulier, le compteur de doses électronique 1000, peut être assemblé dans la poignée 20. Ce compteur 1000 peut notamment détecter les déplacements du réservoir 100, par exemple au moyen d'un coulisseau 1010' qui est déplacé par le réservoir 100 ou par l'élément de blocage 500", lorsqu'ils arrivent en position de distribution. En variante, le compteur 1000 pourrait être relié à un capteur, notamment un capteur à membrane, qui détecte la distribution de la dose de produit fluide, par exemple dans le puits de soupape 700. D'autres moyens d'actionner le compteur électronique 1000 sont aussi possibles, par exemple la détection du déplacement de la soupape 210 de la valve doseuse par rapport au corps de valve 201.

De préférence, le dispositif comporte aussi des moyens d'émission de signaux 1100 pour communiquer à distance notamment des informations relatives aux actionnements du dispositif. En particulier, le corps 10 et/ou la poignée 20 peut comporter un module d'émission de signaux, pour communiquer à distance avec une base quelconque. Des moyens d'alimentation appropriés sont avantageusement prévus.

Dans l'exemple représenté sur les figures 8 à 10, les modules électroniques 1000, 1100 ainsi que le coulisseau associé 1010' sont placés dans la poignée 20, mobile par rapport au corps 10. Mais en variante, on peut imaginer des modules fixes par rapport au corps 10.

La présente invention est notamment applicable pour le traitement des crises d'asthme ou de BPCO (Broncho Pneumopathie Obstructive) en utilisation avec des formulations de type salbutamol, aclidinium, formoterol, tiotropium, budesonide, fluticasone, indacaterol, glycopyrronium, salmeterol, umeclidinium bromide, vilanterol, olodaterol, striverdi, ou toute combinaison de ces formulations.

La présente invention a été décrite en référence à plusieurs modes de réalisation et variantes avantageux, mais il est entendu qu'un homme du métier peut y apporter toutes modifications, sans sortir du cadre de la présente invention tel que défini par les revendications annexées.

## Revendications

1. Dispositif de distribution de produit fluide synchronisé avec l'inhalation, comportant un corps (10; 10') pourvu d'un embout buccal (400), un réservoir de produit (100) contenant un produit fluide et un gaz propulseur étant monté axialement coulissant dans ledit corps (10; 10'), une valve doseuse (200) comportant une soupape (210) étant assemblée sur ledit réservoir (100) pour distribuer sélectivement le produit fluide, **caractérisé en ce que** ledit dispositif comporte:
- un élément d'actionnement (500, 500', 500"; 550) déplaçable et/ou déformable entre une position de non actionnement, dans laquelle ladite valve doseuse (200) ne peut pas être actionnée, et une position d'actionnement, dans laquelle ladite valve doseuse (200) peut être actionnée,
- un système de déclenchement commandé par l'inhalation comportant un organe sensible à l'inhalation (60, 61; 65, 66), déformable et/ou déplaçable sous l'effet de l'inhalation, ledit organe sensible à l'inhalation (60, 61; 65, 66), lorsqu'il se déforme et/ou se déplace, déplaçant et/ou déformant ledit élément d'actionnement (500, 500', 500"; 550) de sa position de non actionnement vers sa position d'actionnement,
- un compteur de doses électronique (1000), et
- des moyens d'émission de signaux (1100) pour communiquer à distance notamment des informations relatives aux actionnements du dispositif.

2. Dispositif selon la revendication 1, dans lequel ledit élément d'actionnement est un élément de blocage (500, 500', 500") qui, en position de non actionnement, coopère d'une part avec le corps (10; 10') et d'autre part avec le réservoir (100) pour empêcher le déplacement axial dudit réservoir (100) dans le corps (10; 10').

3. Dispositif selon la revendication 2, comportant un élément déclencheur (600, 600') déplaçable et/ou déformable entre une position de verrouillage, dans laquelle il bloque ledit élément de blocage (500, 500") dans sa position de blocage, et une position de libération, dans laquelle il ne bloque pas ledit élément de blocage (500, 500").

4. Dispositif selon la revendication 3, dans lequel ledit système de déclenchement commandé par l'inhalation comporte une membrane déformable (61) définissant une chambre d'air déformable (60), ladite membrane déformable (61) étant fixée audit élément déclencheur (600, 600'), ladite membrane déformable (61) étant déformée lors de l'inhalation de telle sorte qu'elle déplace ledit élément déclencheur (600, 600') de sa position de blocage vers sa position de libération.

5. Dispositif selon la revendication 3 ou 4, dans lequel ledit élément déclencheur (600, 600') est accessible manuellement par l'utilisateur pour pouvoir être déplacé manuellement vers sa position de libération même en l'absence d'inhalation.

6. Dispositif selon l'une quelconque des revendications précédentes, dans lequel un organe d'actionnement (800) est assemblé sur le réservoir (100) du côté axialement opposé à ladite valve doseuse (200), ledit organe d'actionnement (800) comportant un manchon creux (801) déplaçable axialement par rapport audit réservoir (100) entre une position de repos et une position chargée, un ressort (850) étant disposé entre le fond du réservoir (100) et le bord supérieur fermé dudit manchon creux (801), de sorte que lorsque l'utilisateur appuie manuellement sur ledit organe d'actionnement (800) pour le déplacer vers sa position chargée, ledit ressort (850) est comprimé, pour transmettre une force axiale (F) audit réservoir (100).

7. Dispositif selon la revendication 6, dans lequel une poignée d'actionnement latérale (20) est montée pivotante sur le corps (10; 10') entre une position de repos et une position d'utilisation dans laquelle elle déplace axialement ledit organe d'actionnement (800) dans sa position chargée.

8. Dispositif selon l'une quelconque des revendications précédentes, dans lequel ledit corps (10; 10') comporte une ouverture (13) reliant l'embout buccal (400) avec l'intérieur du corps (10), ladite ouverture (13) étant fermée en début d'inhalation par un clapet (14), de sorte que le flux d'inhalation est initialement principalement transmis au système de déclenchement par l'inhalation.

9. Dispositif selon la revendication 8, dans lequel ledit clapet (14) est ouvert lorsque ledit élément de blocage (500") se déplace vers sa position d'actionnement.

10. Dispositif selon la revendication 2, dans lequel ledit élément de blocage (500') comporte une extension axiale (505) dont une extrémité inférieure (506) est fixée radialement et axialement par rapport audit corps (10) et une extrémité supérieure coopère avec ledit réservoir (100) en position de non actionnement.

11. Dispositif selon la revendication 10, dans lequel ledit système de déclenchement commandé par l'inhalation comporte un piston (65) coulissant dans une chambre (66) entre une position de repos et une position d'inhalation.

12. Dispositif selon la revendication 11, dans lequel ledit élément de blocage (500') est assemblé sur une tige (540) solidaire du piston (65), de sorte que lors de l'inhalation, ladite tige (540) se déplace radialement déformant et/ou déplaçant ladite extension axiale (505) vers sa position d'actionnement.

13. Dispositif selon la revendication 1, dans lequel ledit élément d'actionnement est un élément de verrouillage (550) qui, en position de non actionnement, permet le déplacement axial de ladite soupape (210) de la valve doseuse (200) ensemble avec ledit réservoir (100) dans le corps (10), empêchant l'actionnement de ladite valve doseuse (200) lorsque ledit réservoir (100) est déplacé axialement dans le corps (10) sans inhalation.

14. Dispositif selon la revendication 13, dans lequel, lors de l'inhalation, ledit élément de verrouillage (550) est déplacé et/ou déformé de telle sorte qu'il bloque le déplacement axial de la soupape (210) par rapport au corps (10).

15. Dispositif selon la revendication 14, dans lequel ledit système de déclenchement commandé par l'inhalation comporte un piston (65) coulissant dans une chambre (66) entre une position de repos et une position d'inhalation.

16. Dispositif selon la revendication 15, dans lequel ledit élément de verrouillage (550) est solidaire d'une tige (540) solidaire du piston (65), de sorte que lors de l'inhalation, ladite tige (540) se déplace radialement déplaçant ledit élément de verrouillage (550) vers sa position d'actionnement dans laquelle il empêche le déplacement axial de ladite soupape (210) de la valve doseuse (200) lorsque ledit réservoir (100) est déplacé axialement dans le corps (10).

## Patentansprüche

1. Vorrichtung zur inhalationssynchronisierten Abgabe eines flüssigen Produkts, umfassend einen Körper (10; 10'), der mit einem Mundstück (400) versehen ist, einen Produkttank (100), der ein flüssiges Produkt und ein Treibgas enthält, und der axial verschiebbar in dem Körper (10; 10') angebracht ist, ein Dosierventil (200), umfassend ein Ventil (210), das auf dem Tank (100) befestigt ist, um das flüssige Produkt selektiv abzugeben, **dadurch gekennzeichnet, dass** die Vorrichtung umfasst:
- ein Betätigungselement (500, 500', 500"; 550), das zwischen einer Nichtbetätigungsposition, in der das Dosierventil (200) nicht betätigbar ist, und einer Betätigungsposition, in der das Dosierventil (200) betätigbar ist, bewegbar und/oder verformbar ist,
- ein inhalationsgesteuertes Auslösesystem, umfassend ein inhalationsempfindlichen Element (60, 61; 65, 66), das durch die Inhalation verformbar und/oder bewegbar ist, wobei das inhalationsempfindliche Element (60, 61; 65, 66) bei Verformung und/oder Bewegung das Betätigungselement (500, 500', 500"; 550) aus seiner Nichtbetätigungsposition in seine Betätigungsposition bewegt und/oder verformt wird,
- einen elektronischen Dosiszähler (1000), und
- Signalübertragungseinrichtungen (1100) zum Fernübermitteln insbesondere von Informationen, die sich auf die Betätigungen der Vorrichtung beziehen.

2. Vorrichtung nach Anspruch 1, wobei das Betätigungselement ein Blockierelement (500, 500', 500") ist, das, in Nichtbetätigungsposition, einerseits mit dem Körper (10; 10') und andererseits mit dem Tank (100) zusammenwirkt, um die axiale Bewegung des Tanks (100) in dem Körper (10; 10') zu verhindern.

3. Vorrichtung nach Anspruch 2, umfassend ein Auslöseelement (600, 600'), das zwischen einer Verriegelungsposition, in der es das Blockierelement (500, 500") in seiner Blockierposition blockiert, und einer Freigabeposition, in er es das Blockierelement (500, 500") nicht blockiert, bewegbar und/oder verformbar ist.

4. Vorrichtung nach Anspruch 3, wobei das inhalationsgesteuerte Auslösesysteme eine verformbare Membran (61) umfasst, die eine verformbare Luftkammer (60) definiert, wobei die verformbare Membran (61) an dem Auslöseelement (600, 600') befestigt ist, wobei die verformbare Membran (61) während der Inhalation derart verformt wird, dass sie das Auslöseelement (600, 600') von seiner Blockierposition in seine Freigabeposition bewegt.

5. Vorrichtung nach Anspruch 3 oder 4, wobei das Auslöseelement (600, 600') für den Benutzer manuell zugänglich ist, um auch ohne Inhalation manuell in seine Freigabeposition bewegt werden zu können.

6. Vorrichtung nach einem der vorhergehenden Ansprüche, wobei das Betätigungselement (800) auf dem Tank (100) auf der dem Dosierventil (200) axial gegenüberliegenden Seite befestigt ist, wobei das Betätigungselement (800) eine hohle Hülse (801) umfasst, die in Bezug auf den Tank (100) zwischen einer Ruheposition und einer geladenen Position axial bewegbar ist, wobei eine Feder (850) zwischen dem Boden des Tanks (100) und dem geschlossenen oberen Rand der hohlen Hülse (801) derart angeordnet ist, dass, wenn der Benutzer manuell auf das Betätigungselement (800) drückt, um es in seine geladene Position zu bewegen, die Feder (850) zusammengedrückt wird, um eine axiale Kraft (F) auf den Tank (100) zu übertragen.

7. Vorrichtung nach Anspruch 6, wobei ein seitlicher Betätigungsgriff (20) schwenkbar zwischen einer Ruheposition und einer Betriebsposition auf dem Körper (10; 10') montiert, in der er das Betätigungselement (800) axial in seine geladene Position bewegt.

8. Vorrichtung nach einem der vorhergehenden Ansprüche, wobei der Körper (10; 10') eine Öffnung (13) umfasst, die das Mundstück (400) mit dem Inneren des Körpers (10) verbindet, wobei die Öffnung (13) zu Beginn der Inhalation mit einer Klappe (14) derart verschlossen ist, dass der Inhalationsstrom anfangs hauptsächlich auf das inhalationsbedingte Auslösesystem übertragen wird.

9. Vorrichtung nach Anspruch 8, wobei die Klappe (14) geöffnet wird, wenn das Blockierelement (500") in seine Betätigungsposition bewegt wird.

10. Vorrichtung nach Anspruch 2, wobei das Blockierelement (500') eine axiale Verlängerung (505) umfasst, wovon ein unteres Ende (506) in Bezug auf den Körper (10) radial und axial befestigt ist und ein oberes Ende mit dem Tank (100) in Nichtbetätigungsposition zusammenwirkt.

11. Vorrichtung nach Anspruch 10, wobei das inhalationsgesteuerte Auslösesystem einen Kolben (65) umfasst, der in einer Kammer (66) zwischen einer Ruheposition und einer Inhalationsposition verschiebbar ist.

12. Vorrichtung nach Anspruch 11, wobei das Blockierelement (500') an einer Stange (540) befestigt ist, die an dem Kolben (65) derart befestigt ist, dass sich die Stange (540) während der Inhalation radial bewegt, wobei sie die axiale Verlängerung (505) in ihre Betätigungsposition verformt und/oder bewegt.

13. Vorrichtung nach Anspruch 1, wobei das Betätigungselement ein Verriegelungselement (550) ist, das, in der Nichtbetätigungsposition, die axiale Bewegung des Ventils (210) des Dosierventils (200) zusammen mit dem Tank (100) in dem Körper (10) ermöglicht, und dabei die Betätigung des Dosierventils (200) verhindert, wenn der Tank (100) in dem Körper (10) ohne Inhalation axial bewegt wird.

14. Vorrichtung nach Anspruch 13, wobei das Verriegelungselement (550) während der Inhalation derart bewegt und/oder verformt wird, dass es die axiale Bewegung des Ventils (210) in Bezug auf den Körper (10) blockiert.

15. Vorrichtung nach Anspruch 14, wobei das inhalationsgesteuerte Auslösesystem einen Kolben (65) umfasst, der in einer Kammer (66) zwischen einer Ruheposition und einer Inhalationsposition verschiebbar ist.

16. Vorrichtung nach Anspruch 15, wobei das Verriegelungselement (550) an einer Stange (540) befestigt ist, die an dem Kolben (65) derart befestigt ist, dass sich die Stange (540) während der Inhalation radial bewegt, wobei das Verriegelungselement (550) in seine Betätigungsposition bewegt wird, in der es verhindert, dass das Ventil (210) des Dosierventils (200) axial bewegt wird, wenn der Tank (100) axial in dem Körper (10) bewegt wird.

## Claims

1. An inhalation-synchronized fluid dispenser device comprising a body (10; 10') provided with a mouthpiece (400), a fluid reservoir (100) containing a fluid and a propellant gas being mounted to slide axially in said body (10; 10'), a metering valve (200) including a valve member (210) being assembled on said reservoir (100) for selectively dispensing the fluid, said device being **characterized in that** it further comprises:
• an actuator element (500, 500', 500"; 550) movable and/or deformable between a non-actuation position, in which said metering valve (200) cannot be actuated, and an actuation position in which said metering valve (200) can be actuated,
• an inhalation-controlled trigger system including an inhalation-sensitive member (60, 61; 65, 66) that is deformable and/or movable under the effect of inhaling, said inhalation-sensitive member (60, 61; 65, 66), when it deforms and/or moves, moving and/or deforming said actuator element (500, 500', 500"; 550) from its non-actuation position towards its actuation position,
• an electronic dose counter (1000), and
• signal-transmitter means (1100) for remotely communicating, in particular information relating to the actuations of the device.

2. A device according to claim 1, wherein said actuator element is a blocking element (500, 500', 500") that, in the non-actuation position, co-operates firstly with the body (10; 10') and secondly with the reservoir (100) so as to prevent said reservoir (100) from moving axially in the body (10; 10').

3. A device according to claim 2, including a trigger element (600, 600') that is movable and/or deformable between a locking position in which it blocks said blocking element (500, 500") in its blocking position, and a release position, in which it does not block said blocking element (500, 500")**.**

4. A device according to claim 3, wherein said inhalation-controlled trigger system includes a deformable membrane (61) definig a deformable air chamber (60), said deformable membrane (61) being fastened to said trigger element (600, 600'), said deformable membrane (61) being deformed during inhaling so that she moves said trigger element (600, 600') from its blocking position towards its release position.

5. A device according to claim 3 or claim 4, wherein said trigger element (600, 600') is accessible manually by the user so that it can be moved manually towards its release position even in the absence of inhaling.

6. A device according to any preceding claim, wherein an actuator member (800) is assembled on the reservoir (100) on the side axially opposite from said metering valve (200), said actuator member (800) comprising a hollow sleeve (801) axially movable relative to said reservoir (100) between a rest position and a primed position, a spring (850) being arranged between the bottom of the reservoir (100) and the closed top edge of said hollow sleeve (801), such that when the user presses manually on said actuator member (800) so as to move it towards its primed position, said spring (850) is compressed, so as to transmit an axial force (F) to said reservoir (100).

7. A device according to claim 6, wherein a laterally-actuated pusher (20) is mounted to pivot on the body (10; 10') between a rest position, and a working position in which it axially moves said actuator member (800) into its primed position.

8. A device according to any preceding claim, wherein said body (10; 10') includes an opening (13) that connects the mouthpiece (400) with the inside of the body (10), said opening (13) being closed at the start of inhaling by a check valve (14), such that the inhalation flow is initially mainly transmitted to the trigger system by inhaling.

9. A device according to claim 8, wherein said check valve (14) is opened when said blocking element (500") moves towards its actuation position.

10. A device according to claim 2, wherein said blocking element (500') comprises an axial extension (505) having a bottom end (506) that is fastened radially and axially relative to said body (10) and a top end that co-operates with said reservoir (100) in the non-actuation position.

11. A device according to claim 10, wherein said inhalation-controlled trigger system includes a piston (65) sliding in a chamber (66) between a rest position and an inhaling position.

12. A device according to claim 11, wherein said blocking element (500') is assembled on a rod (540) secured to the piston (65), so that during inhaling, said rod (540) moves radially, deforming and/or moving said axial extension (505) towards its actuation position.

13. A device according to claim 1, wherein said actuator element is a locking element (550) that, in its non-actuation position, enables said valve member (210) of the metering valve (200) together with said reservoir (100) in the body (10), preventing the actuation of the said metering valve (200) when said reservoir (100) is moved axially in the body (10) without inhaling.

14. A device according to claim 13, wherein, during inhaling, said locking element (550) is moved and/or deformed so that it prevents the moving axially of the valve member (210) relative to the body (10).

15. A device according to claim 14, wherein said inhalation-controlled trigger system includes a piston (65) sliding in a chamber (66) between a rest position and an inhaling position.

16. A device according to claim 15, wherein said locking element (550) is secured to a rod (540) secured to the piston (65), so that during inhaling, said rod (540) moves radially, moving said locking element (550) towards its actuation position in which it prevents the moving axially of said valve member (210) of the metering valve (200) when said reservoir (100) is moved axially in the body (10).
